Europäisches Patentamt

European Patent Office

Office européen des brevets

(19)

(11) Veröffentlichungsnummer: **0 177 768**
**B1**

(12)

# EUROPÄISCHE PATENTSCHRIFT

(45) Veröffentlichungstag der Patentschrift:
12.08.87

(51) Int. Cl.⁴: **C 07 C 31/30, C 07 C 29/70**

(21) Anmeldenummer: **85111278.9**

(22) Anmeldetag: **06.09.85**

(54) Verfahren zur katalytischen Herstellung von Alkalialkoholaten.

(30) Priorität: **10.10.84 DE 3437152**

(43) Veröffentlichungstag der Anmeldung:
**16.04.86 Patentblatt 86/16**

(45) Bekanntmachung des Hinweises auf die Patenterteilung:
**12.08.87 Patentblatt 87/33**

(84) Benannte Vertragsstaaten:
**AT BE CH DE FR GB IT LI LU NL SE**

(56) Entgegenhaltungen:
**DE-C-845 341**
**GB-A-763 266**
**US-A-2 069 403**
**US-A-2 336 045**

(73) Patentinhaber: **DYNAMIT NOBEL AKTIENGESELLSCHAFT, Postfach 1261, D-5210 Troisdorf, Bez. Köln (DE)**

(72) Erfinder: **Auschner, Reinhard, Max- Ernst- Strasse 17, D-5210 Troisdorf 16 (DE)**
Erfinder: **Schmittinger, Peter, Dr., Germanenstrasse 42, D-5216 Niederkassel 6 (DE)**
Erfinder: **Stephan, Rudolf, Dr., Lessingstrasse 5, D-5210 Troisdorf- Sieglar (DE)**

## Beschreibung

Die Erfindung betrifft ein Verfahren zur katalytischen Herstellung von Alkalialkoholaten aus Alkaliamalgamen und Alkoholen.

Alkalialkoholate, besonders solche, deren Alkoholkomponente bis zu 4 Kahlenstoffatome enthält, sind wertvolle Chemikalien. Sie werden z. B. als Katalysatoren bei der Synthese vieler organischer Verbindungen verwendet. Dabei haben vorzugsweise die Alkohalate des Natriums und des Kaliums praktische Bedeutung erlangt. Zur Darstellung von Alkalialkoholaten sind mehrere Methoden bekannt (F.A. Dickes, Ber. Dtsch.Chem.Ges. 63, 2753 [1930]). So enthalten Lösungen von Alkalihydroxiden in einem Alkohol Alkalialkohalat im Gleichgewicht. Durch z. B. destillative Entfernung des in diesem Gleichgewicht befindlichen Wassers gelangt man zu reinen Alkalialkahalaten. Besonders bei niedrig siedenden Alkahalen wird für diese Art der Gleichgewichtsverschiebung jedach sehr viel Energie benötigt.

Auf direktem Wege kommt man zu reinem Alkalialkoholaten, indem man ein Alkalimetall in dem entsprechenden Alkohol auflöst. Dabei reagieren Natrium und Kalium mit niederen aliphatischen Alkoholen, wie Methanal und Äthanol, stürmisch unter Wasserstoffentwicklung. Höhere Alkohole, wie z. B. die Propanole und Butanole, setzt man vorzugsweise oberhalb des Schmelzpunktes der Alkalimetalle, gegebenenfalls unter Druck und Rühren, mit diesen um. Die Methode der direkten Herstellung der Alkalialkoholate aus Metall und Alkohol kommt für ein kommerzielles Verfahren kaum in Frage, weil die hierfür als Ausgangsmaterialien benötigten Alkalimetalle zu teuer sind.

Wirtschaftlicher ist es, als Alkaliquelle das flüssige, bei der Chlaralkali-Elektrolyse nach dem Quecksilber-Verfahren anfallende Alkaliamalgam einzusetzen.

Die Umsetzung von Alkaliamalgam mit Alkoholen sowie die Verwendung von Katalysatoren für diese Reaktion sind bekannt.

R.B. Mac Mullin, Chemical Engineering Progress, Sept.1950 S. 440, erwähnt unter anderem die Umsetzung von Alkaliamalgam mit Methanol in einem Reaktor, welcher Graphit als Kontakt enthält. In den US-PS 2 336 045 und 2 761 880 werden nicht amalgamierende Stoffe, wie z. B. Eisen, Graphit oder Mischungen davon, als Kontakt vorgeschlagen.

In der US-PS 2 069 403 werden als Katalysatoren auch Metallgitter, bestehend aus Schwermetallegierungen, beschrieben.

Es bestand die Aufgabe, ein Verfahren zu entwickeln, welches folgende Anforderungen erfüllt:

Die Reaktion zwischen dem Amalgam und dem Alkohol sollte mit möglichst hoher Geschwindigkeit ablaufen, um in einem technischen Reaktor hohe Raum-Zeit-Ausbeuten zu erreichen Ferner sollte nach dieser Reaktion die Konzentration des Alkali-Metalls im Amalgam möglichst gering sein, da in einer zweiten Reaktionsstufe der Restgehalt an Alkalimetall mit Wasser zu Alkalihydroxidlösung umgesetzt werden muß, welche kein Zielprodukt des vorliegenden Verfahrens ist. Diese Umsetzung des Amalgams mit Wasser ist notwendig, um das im Amalgam enthaltene Alkalimetall vollständig umzusetzen. Anderenfalls würde bei der Rückführung des Quecksilbers in die Elektrolysezelle die Alkalimetallkonzentration zu hoch und das Amalgam fest werden. Das Verfahren sollte kontinuierlich durchführbar sein.

Diese Aufgabe wurde gemäß dem Kennzeichen des Anspruchs 1 gelöst.

Es wurde gefunden, daß die Zersetzung von Alkaliamalgam durch Alkohole überraschenderweise besonders gut durch Schwermetalloxide und Mischungen daraus, welche in feiner Verteilung auf die Oberfläche von Anthrazit aufgebracht werden, katalysiert wird. Dabei hat der Anthrazit gegenüber dem bereits beschriebenen Graphit den Vorteil einer besseren Benetzung durch die flüssigen Reaktanden Amalgam und Alkohol. In einem kontinuierlichen Reaktor, in welchem die Reaktanden nach dem Gegenstromprinzip am Kontakt möglichst fein verteilt in Berührung kommen sollen, ist die Benetzung des Katalysators van Wichtigkeit, da es sich im vorliegenden Fall um eine Reaktion zwischen zwei flüssigen Phasen und einer festen Phase handelt.

Der Anthrazit ist außerdem weitgehend abriebfest. Seine katalytische Wirkung wird wesentlich dadurch erhöht, daß auf seiner Oberfläche Oxide oder Oxidgemische von Schwermetallen aufgebracht werden.

Gegenüber der bekannten Anwendung von Schwermetallen und deren Legierungen als Katalysatoren in Form von Blechen, Netzen oder Spänen erhält man auf diese Weise eine große Oberfläche an katalytisch wirksamer Substanz und benötigt von ihr nur geringe Mengen, da nur die Oberfläche des Trägermaterials Anthrazit mit den Metalloxiden belegt ist.

Besonders die Kombination der Oxide von Nickel und Molybdän gemäß Anspruch 2 zeichnet sich durch eine hohe Aktivität aus.

Nach Anspruch 3 werden beim erfindungsgemäßen Verfahren vorzugsweise aliphatische Alkohole mit 1 bis 4 Kohlenstoffatomen umgesetzt. Es können jedoch auch andere Alkohole bei diesem Verfahren als Ausgangsmaterial verwendet werden, wie z. B. aliphatische Alkohole mit mehr als 4 Kohlenstoffatomen.

Die Erfindung wird nachstehend anhand der Beispiele näher erläutert.

## Beispiel 1

(Herstellung des Katalysators)

In 500 l Wasser werden 50 kg Nickel(II)-acetat-tetrahydrat und 10 kg Natriummolybdat-dihydrat bei 95°C gelöst. In diese heißgesättigte Lösung gibt man 500 kg Anthrazit-Nuß stückig, ein und läßt während ca. 24 Stunden auf 20°C abkühlen. Die Lösung wird abgezogen, das imprägnierte Material wird getrocknet und in einem Ofen, z. B. einem Muffeloder Tunnelofen, auf 400 bis 1200°C erhitzt. Anschließend läßt man abkühlen und siebt die Feinanteile (Korngröße unterhalb 4 mm) ab.

Bei dieser Behandlung laufen folgende Vorgänge ab:

Auf dem harten Trägermaterial lagert sich durch Ausfällung aus der heißgesättigten Metallsalzlösung beim Wiederabkühlen Metallsalz ab, welches sich bei Temperaturen von 400 bis 1200°C in die entsprechenden Metalloxide umwandelt, sintert und nach dem Sintervorgang fest auf dem Trägermaterial haftet.

**Beispiele 2 bis 5**

Zwecks Beurteilung der Aktivität der Katalysatorproben wurden in einem Modellreaktor 100 g Natriumamalgam mit 0,3 Gew.-% Natrium vorgelegt und 10 g Katalysatorkörner zugefügt. Darüber wurden 100 ml Methanol geschichtet. Sowohl das Amalgam als auch der Alkohol wurden gleichzeitig mit dem selben Rührer gerührt. Als Maß für die Aktivität diente die zeitliche Zunahme der Leitfähigkeit der sich bildenden alkoholischen Alkoholatlösung bei 60°C.

In diesem Modellreaktor ergaben sich für verschiedene Katalysatoren folgende Zeitspannen bis zur vollständigen Zersetzung des Amalgams.

|  | Katalysator | Reaktionszeit mit Methanol (Minuter) |
|---|---|---|
| Beispiel 2: | Zersetzergraphit | 57,5 ± 3 |
| Beispiel 3: | Anthrazit-Nuß unbehandelt | 44,3 ± 3 |
| Beispiel 4: | Anthrazit-Nuß mit Nickel(II)-oxid aktiviert | 18,5 ± 1 |
| Beispiel 5: | Anthrazit-Nuß mit einer Mischung von Nickel(II)-oxid und Molybdän(VI)-oxid aktiviert | 5,5 ± 0,3. |

Die beste Zersetzungsleistung ergibt sich mit dem nach oben beschriebener Darstellung hergestellten Katalysator gemäß Beispiel 5.

**Beispiele 6 bis 9**

In weiteren Versuchsserien wurden analog den Beispielen 2 bis 5 die Reaktionszeiten mit längerkettigen Alkoholen bestimmt.

Gegenüber den Beispielen 2 bis 5 verlängern sich die Reaktionszeiten
- bei Einsatz von Ethanol um den Faktor 2 (Beispiel 6)
- bei Einsatz von 1-Propanol um den Faktor 5 (Beispiel 7)
- bei Einsatz von 1-Butanol um den Faktor 14 (Beispiel 8)

Sekundäre Alkohole reagieren deutlich schlecher, zum Beispiel bei Einsatz von 2-Butanol um den Faktor 23 (Beispiel 9).

**Patentansprüche**

1. Verfahren zur katalytischen Herstellung von Alkalialkoholaten aus Alkaliamalgamen und Alkoholen, gekennzeichnet durch Verwendung eines Katalysators aus stückigem Anthrazit als Trägermaterial, dessen Oberfläche mit einem Schwermetalloxid oder Schwermetalloxidmischungen belegt ist.

2. Verfahren nach Anspruch 1, dadurch gekennzeichnet, daß als Schwermetalloxidmischung eine Mischung aus Nickel- und Molybdänoxid eingesetzt wird.

3. Verfahren nach einem der Ansprüche 1 oder 2, dadurch gekennzeichnet, daß als Alkoholkomponente aliphatische Alkohole mit 1 bis 4 Kohlenstoffatomen umgesetzt werden.

**Claims**

1. Process for the catalytic production of alkali alcoholates from alkali amalgams and alcohols, characterised by use of a catalyst formed of lumps of anthracite as support material with its surface coated with a heavy metal oxide or heavy metal oxide mixtures.

2. Process according to claim 1, characterised in that a mixture of nickel oxide and molybdenum oxide is employed as heavy metal oxide mixture.

3. Process according to one of claims 1 or 2, characterised in that aliphatic alcohols with 1 to 4 carbon atoms are reacted as alcohols components.

**Revendications**

1. Procédé pour préparer catalytiquement des alcoolates de métaux alcalins à partir d'amalgames de métaux alcalins et d'alcools, caractérisé par l'utilisation d'un catalyseur formé d'anthracite en morceaux comme matière de support, dont la surface est revêtue d'un oxyde de métal lourd ou de mélanges d'oxydes de métaux lourds.

2. Procédé selon la revendication 1, caractérisé en ce qu'on utilise comme mélange d'oxydes de métaux lourds un mélange d'oxydes de nickel et d'oxyde de molybdène.

3. Procédé selon l'une des revendications 1 ou 2, caractérisé en ce qu'on utilise comme composant alcool des alcools aliphatiques comportant 1 à 4 atomes de carbone.